**Europäisches Patentamt**

**European Patent Office**

**Office européen des brevets**

(19)

(11) Veröffentlichungsnummer: **0 247 584**
**B1**

(12) **EUROPÄISCHE PATENTSCHRIFT**

(45) Veröffentlichungstag der Patentschrift: **09.01.91**

(21) Anmeldenummer: **87107677.4**

(22) Anmeldetag: **26.05.87**

(51) Int. Cl.⁵: **A 61 K 47/00,** A 61 K 47/34,
A 61 K 9/10

(54) **Verwendung von Äthylenoxid-Propylenoxid-Blockpolymeren zur Schaumkontrolle in flüssigen Arzneizubereitungen.**

(30) Priorität: **30.05.86 DE 3618217**

(43) Veröffentlichungstag der Anmeldung:
**02.12.87 Patentblatt 87/49**

(45) Bekanntmachung des Hinweises auf die
Patenterteilung:
**09.01.91 Patentblatt 91/02**

(84) Benannte Vertragsstaaten:
**AT BE CH DE ES FR GB GR IT LI LU NL SE**

(56) Entgegenhaltungen:
**DE-C- 925 375**
**GB-A-2 067 533**
**US-A-2 937 104**
**US-A-3 142 628**
**US-A-4 104 033**
**US-A-4 533 542**

**SOAP AND CHEMICAL SPECIALTIES, Band 31,
Nr. 10, Oktober 1955, Seiten 48-67, Mac Nair-
Dorland Co., New York, US; J.W.
McCUTCHEON: "Synthetic detergents and
emulsifiers-up to date"**

(73) Patentinhaber: **HOECHST
AKTIENGESELLSCHAFT
Postfach 80 03 20
D-6230 Frankfurt am Main 80 (DE)**

(72) Erfinder: **Schwabe, Karl-Detlev, Dr.
Mailänder Strasse 16/163
D-6000 Frankfurt am Main (DE)**

**Beschreibung**

Parenteral zu applizierende flüssige Arzneizubereitungen, die zur Schaumbildung neigen, sind schwierig und nur unter Zeitverlust herzustellen und abzufüllen. Auch die Vorbereitung zur Applikation verzögert sich, da das Zusammenbrechen des Schaums abgewartet werden muß. Ein Injektionslösung, die auf der Oberfläche mit Schaum bedeckt ist, kann nicht appliziert werden.

Gebräuchliche Antischaummittel für oral zu applizierende flüssige Arzneizubereitungen, wie Silikonöl oder Oktanol, sind aus toxikologischen Gründen als Zusätze für parenteral anzuwendende Zubereitungen problematisch. Außerdem können diese Zusätze Trübungen in Injektionslösungen verursachen. Es wurde überraschend gefunden, daß Spuren eines Tensids vom Typ Äthylenoxid-Propylenoxid-Blockpolymere einen schnellen Zusammenbruch des Schaums bewirken.

Äthylenoxid-Propylenoxid-Blockpolymere werden als schwach schäumende Rohstoffe in Spül- und Waschmitteln verwendet. Ihre Eigenschaften als Emulgatoren, Emulsionsspalter und als Netzmittel sind beschrieben worden. Jedoch ist ihre Verwendbarkeit als Antischaummittel nicht bekannt. Durch die Anwesenheit von liphophilen Substanzen in aus Kunststoff bestehendem Verpackungsmaterial für Cefazolin-Pulver kommt es bei der Auflösung des Pulvers zu einer Trübung der Lösung. Um diese Trüblöslichkeit zu vermindern wird entsprechend den Angaben in der US-Patentschrift 4533542 Cefazolin z.B. mit Pluronic® überzogen. Eine Methode zur Schaumkontrolle wird nicht erwähnt.

Die Erfingung betrifft daher die Verwendung von Äthylenoxid-Propylenoxid-Blockpolymeren zur Schaumkontrolle in flüssigen Arzneizubereitungen, insbesondere in solchen zurparenteralen Applikation.

Als Äthylenoxid-Propylenoxid-Blockpolymer ist Polyäthylen-Polypropylenglykol 1800 (auch PPG 1800 bezeichnet) besonders bevorzugt. Die Verwendung in parenteral zu applizierenden Zubereitungen ist besonders bevorzugt. Das Antischaummittel muß die Reinheitskriterien, die üblicherweise an Hilfsstoffe für Arzneimittel gestellt werden, erfüllen.

Die Äthylenoxid-Propylenoxid-Blockpolymere eignen sich prinzipiell zur Schaumkontrolle bei allen flüssigen, insbesondere bei wäßrigen Zubereitungen von Arzneimitteln. Selbstverständlich müssen die Arzneistoffe mit dem eingesetzten Äthylenoxid-Propylenoxid-Blockpolymeren compatibel sein. Geeignete Arzneistoffe sind z.B. Cephalosporinderivate wie Cefpirom (HR 810) und Penicillinderivate wie Procain-benzylpenicillin.

Die flüssige Zubereitung enthält etwa 0,1 bis 0,00001 Gew.-% Äthylenoxid-Propylenoxid-Blockpolymere wie z.B. PPG 1800, vorzugsweise 0,01—0,0001 Gew.-% PPG 1800.

Die Zubereitungen lassen sich herstellen, indem man Äthylenoxid-Propylenoxid Blockpolymere auf den festen Wirkstoff aufbringt oder ihn mit dem Polymer imprägniert oder das Polymer dem Lösungsmittel zusetzt.

<div align="center">Beispiel 1</div>

Eine Zubereitung für Injektionsswecke enthaltend 1,23 g Hr 810-sulfat und 0,22 g Na$_2$CO$_3$ (wasserfrei) zeigte nach Lösung in 10 ml Wasser einen Schaum, der ca. 5 Minuten stabil war.

Wird zum Lösen Statt Wasser eine 0,0005 %ige wässrige Lösung von Polyäthylen-Polypropylenglykol 1800 verwendet, so bricht der Schaum praktisch sofort nach der Auflösung zusammen.

Die gleiche Wirkung kann erzielt werden, wenn etwa äquivalente Mengen an PPG 1800 auf einzelne oder alle Festsubstanzkomponenten aufgebracht wird, z.B. durch Fällung in Gegenwart von PPG 1800, durch Zusatz von PPG 1800 zur Nachwaschlösung der gefällten Festsubstanzen, durch Aufsprühen von PPG 1800-Lösung auf die Festsubstanzen mit anschließendere Trocknung. Außerdem läßt sich der Antischaum-effekt von PPG 1800 durch Imprägnierung der Primärpackmittel (z.B. Injektionsflaschen und/oder Injektionsflaschenstopfen) mit PPG 1800 erzielen.

<div align="center">Beispiel 2</div>

Eine Procain-Benzylpenicillin Suspension 300000IE/ml läßt sich wegen zu starken Schäumens schlecht abfüllen. Die durch Schaum volumenmäßig ausgedehnte Suspension paßt nicht mehr in die für die Primärverpackung vorgesehenen Injectionsflaschen. Bereits ein 0,001 %iger Zusatz von PPG 1800 wirkt der Schaumbildung entgegen, wie die nachfolgende Tabelle zeigt:

<div align="center">TABELLE</div>

<div align="center">Dichte (g/ml)</div>

| Suspension | 2 Minuten nach dem Aufschütteln | 60 Minuten nach dem Aufschütteln |
|---|---|---|
| PPG 1800 frei | 1.012 | 1.00 |
| Zusatz von 0,001% PPG 1800 | 1.041 | 1.055 |
| Zusatz von 0.01% PPG 1800 | 1.053 | 1.058 |

<div align="center">2</div>

## EP 0 247 584 B1

### Patentansprüche

1. Verwendung von Äthylenoxid-Propylenoxid-Blockpolymeren zur Schaumkontrolle in flüssigen Arzneizubereitungen.

2. Verwendung von Äthylenoxid-Propylenoxid-Blockpolymeren zur Schaumkontrolle in flüssigen, parenteral zu applizierenden Arzneizubereitungen.

3. Verwendung von Polyethylen-Polypropylenglykol 1800 zur Schaumkontrolle in flüssigen Arzneizubereitungen.

4. Verwendung gemäß einem der Anpsrüche 1 bis 3 Arzneizubereitungen enthaltend ein Cephalosporinderivat, insbesondere Cefpirom, oder ein Penicillinderivat, insbesondere Procain-Benzylpenicillin.

5. Verwendung gemäß einem der Ansprüche 1 bis 4 in einer Konzentration von 0,1 bis 0,00001, insbesondere von 0,01 bis 0,001 Gew.%.

### Revendications

1. Utilisation de polymères séquencés d'oxyde d'éthylèneoxyde de propylène pour le contrôfle de la mousse dans des préparations pharmaceutiques liquides.

2. Utilisation de polymères séquencés d'oxyde d'éthylèneoxyde de propylène pour le contrôle de la mousse dans des préparations pharmaceutiques liquides à usage parentéral.

3. Utilisation de polyéthylène-polypropylèneglycol 1800 pour le contrôle de la mousse das des préparations pharmaceutiques liquides.

4. Utilisation selon une des revendications 1 à 3, dans des préparations pharmaceutiques contenant un dérivé de céphalosporine, en particulier cefpirom, ou un dérivé de pénicilline, en particulier procaïne-benzylpénicilline.

5. Utilisation selon une des revendications 1 à 4, en une concentration de 0,1 à 0,00001, en particulier 0,01 à 0,0001% en poids.

### Claims

1. The use of ethylene oxide/propylene oxide block copolymers for controlling foam in liquid pharmaceutical formulations.

2. The use of ethylene oxide/propylene oxide block copolymers for controlling foam in liquid pharmaceutical formulations for parenteral administration.

3. The use of polyethylene/polypropylene glycol 1800 for controlling foam in liquid pharmaceutical formulations.

4. The use as claimed in one of claims 1 to 3 in pharmaceutical formulations containing a cephalosporin derivative, especially cefpirom, or a penicillin derivative, especially procaine benzylpenicillin.

5. The use as claimed in one of claims 1 to 4 in a concentration of 0.1 to 0.00001, in particular of 0.01 to 0.0001, % by weight.

3